(19) Europäisches Patentamt
European Patent Office
Office européen des brevets

(11) Publication number: **0 337 598**
**A2**

# (12) EUROPEAN PATENT APPLICATION

(21) Application number: **89301726.9**

(51) Int. Cl.⁴: **A61K 31/40**

(22) Date of filing: **22.02.89**

The title of the invention has been amended (Guidelines for Examination in the EPO, A-III, 7.3).

(30) Priority: **26.02.88 JP 45620/88**
**24.08.88 JP 213393/88**

(43) Date of publication of application:
**18.10.89 Bulletin 89/42**

(84) Designated Contracting States:
**DE FR GB IT**

(71) Applicant: **SAWAI PHARMACEUTICAL CO., LTD.**
**4-25, Akagawa 1-chome Asahi-ku**
**Osaka-shi Osaka 535(JP)**

Applicant: **MEDISA SHINYAKU INC.**
**15-9, Honcho 4-Chome Nihonbashi**
**Chuo-Ku Tokyo-to(JP)**

(72) Inventor: **Toya, Harumasa**
**301 Mezon Ueda 5-11-3, Kasuga-Cho**
**Toyonaka-shi Osaka-fu(JP)**
Inventor: **Kato, Yoshiko**
**7-36, Higashiyama-cho Koyoen**
**Nishinomiya-shi Hyogo-ken(JP)**
Inventor: **Asanaka, Miyuki**
**203 Mezondosafuran 2-63, Tenjinmachi**
**Yonago-shi Tottori-ken(JP)**
Inventor: **Kurimura, Takashi C2-1 Tottori Daigaku Igakubu**
**Shukusha 110-1 Uchimachi**
**Yonago-shi Tottori-ken(JP)**

(74) Representative: **Atkinson, Peter Birch et al**
**Marks & Clerk Suite 301 Sunlight House**
**Quay Street**
**Manchester M3 3JY(GB)**

## (54) Use of porphyrins and metalloporphyrins in the treatment of diseases caused by Human Immunodeficiency Viruses.

(57) A use of the compound of the formula;

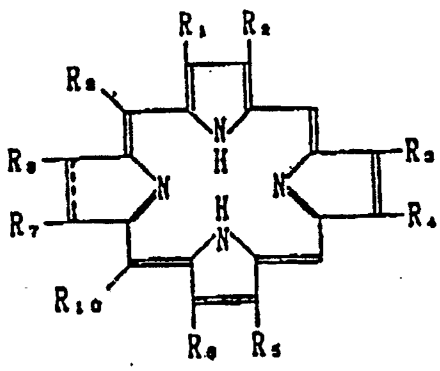

(I)

wherein either $R_1$, $R_2$, $R_3$, $R_4$, $R_5$, $R_6$, $R_7$ and $R_8$ are the same or different, each hydrogen atom, a lower alkyl group, a lower alkenyl group, a hydroxy(lower)alkyl group, a carboxy group or a carboxy(lower)alkyl group,
$R_9$ is hydrogen atom or a carboxy(lower)alkyl group,
with the proviso that at least one of $R_1$, $R_2$, $R_3$, $R_4$, $R_5$, $R_6$, $R_7$, $R_8$ and $R_9$ is a hydroxy(lower)alkyl group, a

EP 0 337 598 A2

carboxy group or a carboxy(lower)alkyl group, and
$R_{10}$ is hydrogen atom or a lower alkyl group, or $R_6$ and $R_{10}$ together form the group $-CO-CH_2-$ and $R_1$, $R_2$, $R_3$, $R_4$, $R_5$, $R_7$, $R_8$ and $R_9$ are as defined above, and
the symbol of a line and a dotted line means a single bond or a double bond,
an N-(lower)alkyl derivative, a metal coordination compound, a physiologically acceptable and hydrolyzable ester or a physiologically acceptable salt thereof for the manufacture of a medicament for treatment of diseases caused by HIVs.

# TREATMENT OF DISEASES CAUSED BY HUMAN IMMUNODEFICIENCY VIRUSES

## BACKGROUND OF THE INVENTION

The present invention relates to the treatment of diseases caused by Human Immunodeficiency Viruses (hereinafter, referred to as HIVs).

HIVs have been known as the causative viruses for AIDS (Acquired Immunodeficiency Syndrome). AIDS is a viral disease in which T-cells are attacked and immunological mechanism is directly destroyed. As the result, opportunistic infections such as pneumocystis carinii pneumonia and candidosis, malignant tumors such as Kaposi sarcoma or mental disorders appear in patients, and once onset of AIDS has occurred, patients die within a short period of half or one year unless any effective therapeutic measure is taken. Therefore, it has been dread as a disease with unfavorable prognosis.

Accordingly, prevalence of AIDS as dangerous disease is becoming a problem of utmost importance on public health as it causes social unrest and other inconvenient situation such as discrimination against patients. The development of a medicine for treating AIDS is thus a matter not only of great importance but also of exceptional urgency.

Examples of medicines presently known to have antiviral effect against HIVs are nucleic acid antiviral agents such as Azidothymidine (AZT) and special plant products such as Glycyrrhizin (from Glycyrrhiza). These medicines, however, have deficiencies in that the nucleic acid antiviral agents have serious toxity and the plant products have not sufficient efficacy. Consequently, there has been a continuous need for anti-HIV agents having sufficient activity and less side effect.

The present inventors have searched for substances having anti-HIV activity within known products having higher safety and successfully found out that Protoporphyrin disodium salt, which has been marketed as hepatic and hence has established safety, has a potent anti-HIV activity and that also some of its analogues have similar activity.

## SUMMARY OF THE INVENTION

In one aspect, the present invention provides a method of treatment for diseases caused by Human Immunodeficiency Viruses (HIVs), which comprises administering, to a human subject in need of such treatment, a therapeutically effective amount of a compound of the formula;

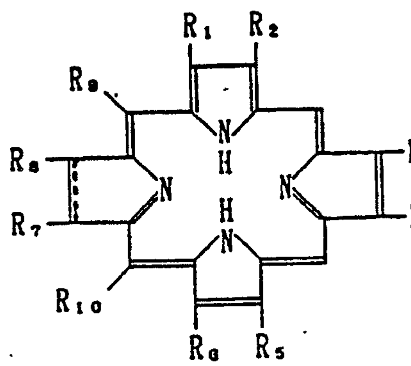

(I)

wherein either $R_1$, $R_2$, $R_3$, $R_4$, $R_5$, $R_6$, $R_7$ and $R_8$ are the same or different, each hydrogen atom, a lower alkyl group, a lower alkenyl group, a hydroxy(lower)alkyl group, a carboxy group or a carboxy(lower)alkyl group,
$R_9$ is hydrogen atom or a carboxy(lower)alkyl group,
with the proviso that at least one of $R_1$, $R_2$, $R_3$, $R_4$, $R_5$, $R_6$, $R_7$, $R_8$ and $R_9$ is a hydroxy(lower)alkyl group, a carboxy group or a carboxy(lower)alkyl group, and
$R_{10}$ is hydrogen atom or a lower alkyl group,
or $R_6$ and $R_{10}$ together form the group $-CO-CH_2-$ and $R_1$, $R_2$, $R_3$, $R_4$, $R_5$, $R_7$, $R_8$ and $R_9$ are as defined above, and
the symbol of a line and a dotted line means a single bond or a double bond,
an N-(lower)alkyl derivative, a metal coordination compound, a physiologically acceptable and hydrolyzable

ester or a physiologically acceptable salt thereof.

In another aspect, the present invention provides a use of the compound of the formula;

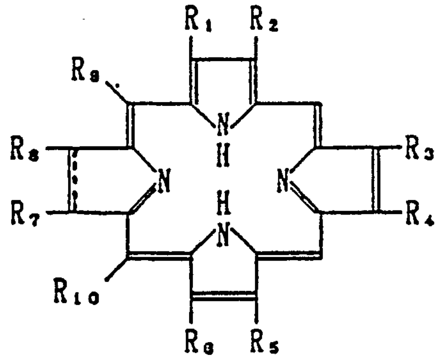

(I)

wherein either $R_1$ $R_2$, $R_3$, $R_4$, $R_5$, $R_6$, $R_7$ and $R_8$ are the same or different, each hydrogen atom, a lower alkyl group, a lower alkenyl group, a hydroxy(lower)alkyl group, a carboxy group or a carboxy(lower)alkyl group,
$R_9$ is hydrogen atom or a carboxy(lower)alkyl group,
with the proviso that at least one of $R_1$, $R_2$, $R_3$, $R_4$, $R_5$, $R_6$, $R_7$, $R_8$ and $R_9$ is a hydroxy(lower)alkyl group, a carboxy group or a carboxy(lower)alkyl group, and
$R_{10}$ is hydrogen atom or a lower alkyl group,
or $R_6$ and $R_{10}$ together form the group $-CO-CH_2-$ and $R_1$, $R_2$, $R_3$, $R_4$, $R_5$, $R_7$, $R_8$ and $R_9$ are as defined above, and
the symbol of a line and a dotted line means a single bond or a double bond,
an N-(lower)alkyl derivative, a metal coordination compound, a physiologically acceptable and hydrolyzable ester or a physiologically acceptable salt thereof for the manufacture of a medicament for treatment of diseases caused by HIVs.

In a further aspect, the present invention provides a pharmaceutical composition comprising the compound of the formula;

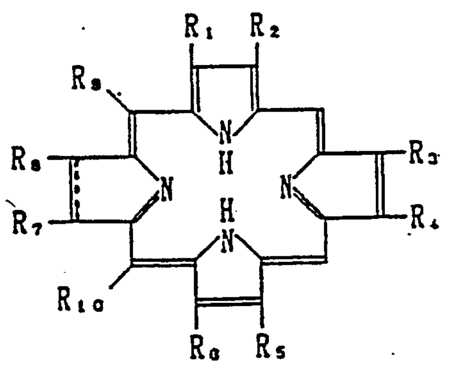

(I)

wherein either $R_1$, $R_2$, $R_3$, $R_4$, $R_5$, $R_6$, $R_7$ and $R_8$ are the same or different, each hydrogen atom, a lower alkyl group, a lower alkenyl group, a hydroxy(lower)alkyl group, a carboxy group or a carboxy(lower)alkyl group,
$R_9$ is hydrogen atom or a carboxy(lower)alkyl group,
with the proviso that at least one of $R_1$, $R_2$, $R_3$, $R_4$, $R_5$, $R_7$, $R_8$ and $R_9$ is a hydroxy(lower)alkyl group, a carboxy group or a carboxy(lower)alkyl group, and
$R_{10}$ is hydrogen atom or a lower alkyl group,
or $R_6$ and $R_{10}$ together form the group $-CO-CH_2-$ and $R_1$, $R_2$, $R_3$, $R_4$, $R_5$, $R_7$, $R_8$ and $R_9$ are as defined above, and
the symbol of a line and a dotted line means a single bond or a double bond,
an N-(lower)alkyl derivative, a metal coordination compound, a physiologically acceptable and hydrolyzable ester or a physiologically acceptable salt thereof as an active ingredient in association with a pharmaceutically acceptable carrier, diluent or excipient.

## DETAILED DESCRIPTION OF THE INVENTION

The terms and definitions described herein are illustrated as follows.

Porphyrins are well known to lie in tautomeric forms. It is to be understood that all the tautomeric forms are regarded as the same and hence included in the scope of the invention and that the above formula is used only for the sake of convenience.

The term "lower" is used to intend a group having up to 6 carbon atoms, unless otherwise provided.

The term "lower alkyl group" includes straight or branched chain saturated hydrocarbon groups having 1 to 6 carbon atoms, such as methyl, ethyl, propyl, isopropyl, butyl, pentyl and hexyl.

The term "lower alkenyl group" includes straight or branched chain unsaturated hydrocarbon groups having 2 to 6 carbon atoms, such as vinyl, allyl, 1-propenyl, butenyl, pentenyl and hexenyl.

The term "hydroxy(lower)alkyl group" includes lower alkyl groups as defined above having more than one and preferably up to three hydroxy groups at any possible positions.

The term "carboxy(lower)alkyl group" includes lower alkyl groups as defined above having more than one and preferably less than three carboxy groups at any possible positions.

Preferred compounds are the compounds of the formula (I), in which either $R_1$, $R_2$, $R_3$, $R_4$, $R_5$, $R_6$, $R_7$ and $R_8$ are, the same or different, each hydrogen atom, methyl, ethyl, vinyl, 1-hydroxyethyl, 3-hydroxypropyl, carboxy, caboxymethyl or 2-carboxyethyl,

$R_9$ is hydrogen atom or caboxymethyl, with the proviso that at least one of them is 1-hydroxyethyl, 3-hydroxypropyl, carboxy, caboxymethyl or 2-carboxyethyl, and $R_{10}$ is hydrogen atom or methyl,

or $R_6$ and $R_{10}$ together form the group $-CO-CH_2-$ and $R_1$, $R_2$, $R_3$, $R_4$, $R_5$, $R_7$, $R_8$ and $R_9$ are as defined above, and

the symbcl of a line and a dotted line is a single bond or a double bond, an N-methyl derivative or a physiologically acceptable salt thereof.

More preferred compounds are the compounds of the formula (I), in which either

$R_1$ is methyl, carboxy, or carboxymethyl,

$R_2$ is hydrogen atom, methyl, ethyl, vinyl, 1-hydroxyethyl, or 2-carboxyethyl,

$R_3$ is methyl, ethyl, carboxymethyl or 2-carboxyethyl,

$R_4$ is hydrogen atom, methyl, ethyl, vinyl, 1-hydroxyethyl, carboxymethyl or 2-carboxyethyl,

$R_5$ is methyl, vinyl, carboxymethyl or 2-carboxyethyl,

$R_6$ is hydrogen atom, methyl, 3-hydroxypropyl, carboxy, carboxymethyl or 2-carboxyethyl

$R_7$ is methyl, 3-hydroxypropyl, carboxymethyl or 2-carboxyethyl,

$R_8$ is methyl, carboxymethyl or 2-carboxyethyl,

$R_9$ is hydrogen atom or carboxymethyl, and

$R_{10}$ is hydrogen atom or methyl,

or $R_6$ and $R_{10}$ together form the group $-CO-CH_2-$ and $R_1$, $R_2$, $R_3$, $R_4$, $R_5$, $R_7$, $R_8$ and $R_9$ are as defined above.

The most preferred compounds are the compounds of the formula (I), in which the symbol of a line and a dotted line is a single bond in the case of Chlorophyllin ligand or a double bond in the other cases, and having the groups shown in the following Table.

Table 1

| Compound | $R_1$ | $R_2$ | $R_3$ | $R_4$ | $R_5$ | $R_6$ | $R_{10}$ | $R_7$ | $R_8$ | $R_9$ |
|---|---|---|---|---|---|---|---|---|---|---|
| Coproporphyrin I | Me | Ce | Me | Ce | Me | Ce | H | Me | Ce | H |
| Coproporphyrin II | Me | Ce | Ce | Me | Me | Ce | H | Ce | Me | H |
| Coproporphyrin III | Me | Ce | Me | Ce | Me | Ce | H | Ce | Me | H |
| Coproporphyrin IV | Me | Ce | Me | Ce | Ce | Me | H | Ce | Me | H |
| Deuteroporphyrin | Me | H | Me | H | Me | Ce | H | Ce | Me | H |
| Hematoporphyrin | Me | He | Me | He | Me | Ce | H | Ce | Me | H |
| Mesoporphyrin | Me | Et | Me | Et | Me | Ce | H | Ce | Me | H |
| Phylloporphyrin | Me | Et | Me | Et | Me | H | Me | Ce | Me | H |
| Protoporphyrin | Me | Vn | Me | Vn | Me | Ce | H | Ce | Me | H |
| Reduced Protoporphyrin | Me | Vn | Me | Vn | Me | Hp | H | Hp | Me | H |

Table 1 (Continued)

| Compound | $R_1$ | $R_2$ | $R_3$ | $R_4$ | $R_5$ | $R_6$ | $R_{10}$ | $R_7$ | $R_8$ | $R_9$ |
|---|---|---|---|---|---|---|---|---|---|---|
| pyrroporphyrin | Me | Et | Me | Et | Me | H | H | Ce | Me | H |
| Rhodoporphyrin | Me | Et | Me | Et | Me | Ca | H | Ce | Me | H |
| Uroporphyrin I | Cm | Ce | Cm | Ce | Cm | Ce | H | Cm | Ce | H |
| Uroporphyrin II | Cm | Ce | Ce | Cm | Cm | Ce | H | Ce | Cm | H |
| Uroporphyrin III | Cm | Ce | Cm | Ce | Cm | Ce | H | Ce | Cm | H |
| Uroporphyrin IV | Cm | Ce | Cm | Ce | Ce | Cm | H | Ce | Cm | H |
| Phytoporphyrin | Me | Et | Me | Et | Me | $-CO-CH_2-$ | | Ce | Me | H |
| Chlorophyllin ligand | Ca | Me | Et | Me | Vn | Me | H | Me | Ce | Cm |

Note: Me = methyl, Et = ethyl, He = 1-hydroxyethyl, Hp = 3-hydroxypropyl, Vn = vinyl, Ca = carboxy, Cm = carboxymethyl, Ce = 2-carboxyethyl, Reduced Protoporphyrin = Di(decarboxyethyl)-di(3-hydroxypropyl)-Protoporphyrin.

While preferred porphyrins used in the invention are naturally occurring isomers, a mixture containing such isomer can also be used in the invention.

The N-(lower)alkyl derivatives of the porphyrins of the formula (I) include compounds wherein at least one of N-atoms has a lower alkyl group as defined above.

The metal coordination compounds of the porphyrins of the formula (I) include those containing, for example, Mg, Fe(II) (e.g. Heme), Fe(III), FeCl (e.g. Hemin), Co, Cu etc. coordinated as a central atom or ion.

The above compounds of formula (I) and the coordination compounds thereof include novel and known compounds. The novel compounds can be prepared by a process similar to that for the known one.

As the porphyrins of the formula (I) are amphoteric, the physiologically acceptable salts thereof include both of the salts with acids and the salts with bases. The term "physiologically acceptable salts" refers to the salts which do not show significant toxity at the doses of administration and have not physical or chemical property that makes the administration difficult. Within such salts, the salts with acids include

those with inorganic (or mineral) acids such as hydrochloric acid, sulfuric acid, phosphoric acid etc., and those with organic acids such as carboxylic acid, e.g. acetic acid, tartaric acid, citric acid, succinic acid, fumaric acid etc. and sulfonic acid, e.g. methanesulfonic acid, ethanesulfonic acid, toluenesulfonic acid etc. The salts with bases include those with inorganic base salt such as alkali metal salts, e.g. sodium salt, potassium salt etc., alkali-earth metal salts, e.g. calcium salt, magnesium salt etc., polyvalent metal salts, e.g. aluminum salt, manganese salt etc. and organic base salts such as organic amine salts, e.g. trimethylamine salt, triethylamine salt, cyclohexylamine salt, ethanolamine salt, diethanolamine salt, tromethamine salt, morpholine salt, piperidine salt, procaine salt, caffeine salt etc. Where plurality of carboxy group are present, any number of said groups may be in the salt form. These salts can be prepared by the conventional process, e.g. by neutralization, salt exchange or ion exchange.

The term "physiologically acceptable and physiologically hydrolyzable ester" refers to the esters which do not show significant toxicity at the doses of administration and have not physical or chemical property that makes the administration difficult, and further, can produce the corresponding acids and alcohols, which do not show significant toxicity at the amount to be produced, by hydrolysis under physiological conditions, for example under conditions within human or animal bodies. Such esters include lower aklyl esters such as methyl ester, ethyl ester, propyl ester, isopropyl ester, t-butyl ester, lower alkanoyloxy(lower)aklyl esters such as acetoxymethyl ester, pivaloyloxymethyl ester, 1-[(lower)alkoxycarbonyloxy]-(lower)alkyl ester such as 1-(ethoxycarbonyloxy)ethyl ester, phthalidyl ester, 5-indanyl ester, 2-(3-phthalidylidene) ethyl ester, (5-methyl-2-oxo-1,3-dioxol-4-yl)methyl ester etc. Where plurality of carboxy groups are present, any number of said groups may be in the ester form. These ester can be prepared by the conventional process, e.g. by condensation between a carboxylic acid or a reactive derivative at the carboxy group thereof with an alcohol or a reactive derivative at the hydroxy groups thereof, by ester exchange etc. These esters may have an improved property such as solubility, stability, absorbablity etc. as compared with the free acids.

The term "HIVS" includes Lymphadenopathy Associated Virus Type 1 (LAV-1) and Type 2 (LAV-2), Human T-Lymphotropic Virus Type III (HTLV-III), AIDS-Associated Retrovirus (ARV) etc., including variant different in property such as infectiosity to cultivated cells.

The term "diseases caused by HIVs" includes AIDS, ARC, PGL and LAS, and in an alternative classification, acute symtoms due to HIV infection, generalized lymphadenopathy, persistent pyrexia, diarrhea, reduction of weight, dementia, bulbar disorder, peripheral nervous disorder, pneumocystis carinii pneumonia, toxoplasmosis, candidosis, Herpesvirus infeciton, Kaposi sarcoma, non-Hodgkin lymphoma, cancer etc.

The term "treatment" includes all kinds of control of disease including prophylaxis and therapy such as prevention of infection, prevention of pathopoiesis where infection is established, prevention of deterioration of disease (sustention), reduction of symptoms, changing to asymptomatic carrier and complete recovery.

For the usages for carrying out the above treatment, the required dose of the porphyrins or salts thereof will, of course, vary depending on age and condition of particular patient, the compound actually used, the mode of administration and treatment desired. In general, however, satisfactory results are obtained in adminstration at a dosage from 10 to 1000 mg, preferably 50 to 500 mg, conveniently administered in 2 to 4 divided doses a day or in sustained release form.

For administration, the porphyrins may be mixed with a pharmaceutical carrier such as organic or inorganic solid or liquid excipient e.g. suitable for local, nasal, peroral, rectal, external administration or injection, and administered in the form of the conventional pharmaceutical preparation. Such preparation includes solids (e.g., tablet, granule, powder, capsule, etc.) and liquids (e.g., liquid, emulsion, suspension, etc.), suppository and ointment. The above carriers include starch, lactose, glucose, sucrose, dextrin, cellulose and derivatives thereof, paraffin, fatty acid glyceride, water, alcohol, gum arabic, etc. If necessary, auxiliary, stabilizer, humectant, emulsifier, lubricant, binder, pH regulating agent, isotonicity agent, and other additives in ordinary use may be added.

The toxicity of typical porphyrins, metal coordination compound, salts or ester thereof used in the invention is known and extremely low. For example, toxicity of Protoporphyrin disodium salt is shown below.

Table 2

| Route of Administration | Animal | $LD_{50}$ (mg/kg) |
|---|---|---|
| intravenous | mouse | 347.3 |
| | rat | 240.3 |
| | rabbit | 220.0 |
| | cat | 215.5 |
| subcutaneous | mouse | 1,147.0 |
| intraperitoneal | mouse | 1,028.6 |
| oral | mouse | > 25,000.0 |

The following Examples will illustrate the present invention in further detail. In the examples, the term "active ingredient" refers to any one of the porphyrins, metal coordination compound, salt or ester used in the invention.

Example 1

| | | |
|---|---|---|
| Active Ingredient | | 20 mg |
| Microcrystalline Cellulose | | 25 mg |
| Hydroxypropyl Cellulose | | 1 mg |
| Carboxymethyl Cellulose Calcium Salt | | 5 mg |
| Magnecium Stearate | | 2 mg |
| Lactose | q.s. to | 150 mg |

The above ingredients are mixed, granulated by wet process, compressed into tablets and sugar-coated according to the conventional method.

Example 2

| | | |
|---|---|---|
| Active Ingredient | | 50 mg |
| Physiological Saline | q.s. to | 10 ml |

Test Example 1

(Preparation of test samples)

Test samples were prepared by diluting samples with RPMI-1640 medium supplemented with 10% fetal calf serum (FCS).

(Assay of anti-HIV activity)

MT-4 cells (MT-4) were challenged with LAV strain of HIV ($10^{2.7}$ median tissue culture infectious dose ($TCID_{50}$/ml) at the multiplicity of infection (m.o.i.) of 0.001 $TCID_{50}$/cell (or 0.01 $TCID_{50}$/cell or 0.1

$TCID_{50}$/cell) for adsorption period of 1 hour, incubated in the presence of test compounds until cytopathic effect (CPE) was observed in control (without the test compounds), and assayed for HIV production. All the procedures were carried out in th dark. Assay was carried out as follows. A microtiter plate (96 wells) was innoculated with MT-4 at $2x10^4$/0.1ml/well, then with the virus suspension obtained above at 0.1 ml/well and observed for CPE during 7 days. Amount of HIV production was shown in terms of $TCID_{50}$/ml.

Compounds were judged as antivirally active when production of HIV was reduced as compared with the control.

Protoporphyrin disodium salt (PPNa) was used as the test compound, sodium dextran sulfate (DSNa), glycyrrhizin (GR) and AZT as the references.

(Results)

The results are shown in the following Table, wherein numerical values indicate $TCID_{50}$/ml. Tox means results were not obtained due to cell toxicity.

Table 3

| | Concentration (μg/ml) | |
|---|---|---|
| | 10 | 1 |
| m.o.i. 0.1 | | |
| PPNa | $10^{3.5}$ | $10^{6.0}$ |
| DSNa | $10^{3.0}$ | $10^{6.0}$ |
| GR | $10^{6.0}$ | $10^{6.0}$ |
| AZT | Tox | $\leq 10^{1.5}$ |
| Control | $10^{6.0}$ | $10^{6.0}$ |
| m.o.i. 0.01 | | |
| PPNa | $10^{3.3}$ | $10^{6.0}$ |
| DSNa | $10^{2.7}$ | $10^{6.0}$ |
| GR | $10^{6.0}$ | $10^{6.0}$ |
| AZT | Tox | $\leq 10^{1.5}$ |
| Control | $10^{6.0}$ | $10^{6.0}$ |

Table 3 (Continued)

| | Concentration ($\mu g/ml$) | |
|---|---|---|
| | 10 | 1 |
| m.o.i. 0.001 | | |
| PPNa | $10^{2.5}$ | $10^{6.0}$ |
| DSNa | $10^{2.0}$ | $10^{6.0}$ |
| GR | $10^{6.3}$ | $10^{5.7}$ |
| AZT | Tox | $\leq 10^{1.5}$ |
| Control | $10^{5.7}$ | $10^{5.7}$ |

Test Example 2

The procedure of Test Example 1 was repeated except that, as the test compounds, hemin (HM), hematoporphyrin disodium (HPNa), Protoporphyrin dimethyl ester (PPMe), Reduced Protoporphyrin (Table 1, PPOH), Coproporphyrin III tetramethyl ester (CPIIIMe), Coproporphyrin III tetrasodium (CPIIINa), Uroporphyrin I dihydrochloride (UPIHCl), Deuteroporphyrin dihydrochloride (DPHCl), Mesoporphyrin dihydrochloride (MPHCl), Phylloporphyrin (Phl), Pyrroporphyrin (PyP), Phytoporphyrin (Pht), N-methylmesoporphyrin* (MeMP), N-methylprotoporphyrin * (MePP), Chlorophyllin ligand(Chl) and Chlorophyl(Ch) were used in place of PPNa.

The results are shown in the following Table, wherein asterisk (*) indicates a mixture of isomers.

Table 4

| | Concentration (μg/ml) | |
|---|---|---|
| | 10 | 1 |
| m.o.i. 0.1 | | |
| HM | $10^{4.2}$ | $10^{6}$ |
| HPNa | $10^{4.0}$ | $10^{6}$ |
| PPMe | $10^{4.2}$ | $10^{6}$ |
| PPOH | $10^{4.2}$ | $10^{6}$ |
| CPIIIMe | $10^{4.3}$ | $10^{6}$ |
| CPIIINa | $10^{4.3}$ | $10^{6}$ |
| UPIHCl | $10^{4.2}$ | $10^{6}$ |
| DPHCl | $10^{4.2}$ | $10^{6}$ |
| MPHCl | $10^{4.2}$ | $10^{6}$ |
| Phl | $10^{4.2}$ | $10^{6}$ |
| PyP | $10^{4.2}$ | $10^{6}$ |
| Pht | $10^{4.2}$ | $10^{6}$ |
| MeMP | $10^{4.2}$ | $10^{6}$ |
| Mepp | $10^{4.2}$ | $10^{6}$ |
| Chl | $10^{4.2}$ | $10^{6}$ |
| Control | $10^{6}$ | $10^{6}$ |

Table 4 (Continued)

| | Concentration (μg/ml) 10 | 1 |
|---|---|---|
| m.o.i. 0.01 | | |
| HM | $10^{4.0}$ | $10^{6}$ |
| HP | $10^{3.8}$ | $10^{6}$ |
| PPMe | $10^{4.0}$ | $10^{6}$ |
| PPOH | $10^{4.0}$ | $10^{6}$ |
| CPIIIMe | $10^{4.1}$ | $10^{6}$ |
| CPIIINa | $10^{4.1}$ | $10^{6}$ |
| UPHCl | $10^{4.0}$ | $10^{6}$ |
| DP | $10^{4.0}$ | $10^{6}$ |
| MP | $10^{4.0}$ | $10^{6}$ |
| Phl | $10^{4.0}$ | $10^{6}$ |
| PyP | $10^{4.0}$ | $10^{6}$ |
| Pht | $10^{4.0}$ | $10^{6}$ |
| Chl | $10^{4.0}$ | $10^{6}$ |
| Ch | $10^{5.0}$ | $10^{6}$ |
| Control | $10^{6}$ | $10^{6}$ |

Table 4 (Continued)

| | Concentration (μg/ml) 10 | 1 |
|---|---|---|
| m.o.i. 0.001 | | |
| HM | $10^{3.2}$ | $10^{6}$ |
| HP | $10^{3.0}$ | $10^{6}$ |
| PPMe | $10^{3.2}$ | $10^{6}$ |
| PPOH | $10^{3.2}$ | $10^{6}$ |
| CPIIIMe | $10^{3.3}$ | $10^{6}$ |
| CPIIINa | $10^{3.3}$ | $10^{6}$ |
| UPHCl | $10^{3.2}$ | $10^{6}$ |
| DP | $10^{3.2}$ | $10^{6}$ |
| MP | $10^{3.2}$ | $10^{6}$ |
| Phl | $10^{3.2}$ | $10^{6}$ |
| PyP | $10^{3.2}$ | $10^{6}$ |
| Pht | $10^{3.2}$ | $10^{6}$ |
| Chl | $10^{3.2}$ | $10^{6}$ |
| Control | $10^{6}$ | $10^{6}$ |

From the above results, it was concluded that the porphyrins used in the invention were antivirally active against HIV.

Test example 3

In view of the disclosure of EP 196 515 A1 in which a therapeutic protein composition suspected to contain viruses was exposed to a light with a divided wave length in the presence of various photosensitizers including porphyrins, some experiments were conducted to see 1) whether or not other photosensitizers than porphyrins have anti-HIV activity in the absence of light and 2) whether or not the porphyrins of the present invention having anti-HIV activity also have antiviral activity against viruses other than HIVs, e.g. against Herpes Simplex Virus (HSV).

1) Test for anti-HIV activity with other photo-sensitizers:

The procedure of Test Example 1 was repeated except that, as the test compounds, Methylene Blue (MB), Quinoline Yellow (QY) and Toluidine Blue (TB) were used in place of PPNa. The results are shown in the following Table.

Table 5

| m.o.i. 0.01 | Concentration (10$\mu$g/ml) |
|---|---|
| MB | $10^6$ |
| QY | $10^6$ |
| TB | $10^6$ |
| Control | $10^6$ |

The above results indicate that they have no antiviral activity.

2) Test for anti-HSV activity with the porphyrins:

Cell and Virus:

Herpes simplex virus (HSV) type 1, strain WT-51, and BS-C-40, an African green monkey kidney cell line, were used. HSV stock was prepared in BS-C-40 and the infectivity of the virus was measured on BS-C-40 monolayers by plaque method. Eagle's minimal essential medium (MEM) supplemented with 8% newborn calf serum was used as the culture medium and cells were incubated at 37°C in a humidified $CO_2$-incubator (5% $CO_2$, 95% air).

Determination of antiviral activity of protoporphyrin:

BS-C-40 monolayers were infected with HSV at the m.o.i. of 0.01 at 37°C for 1 hour and the monolayers were washed with MEM, and fresh culture medium was added. After 18 hours of incubation in the presence or absence of protoporpyrin, the virus infectivity in the culture fluid was measured by plaque method.

Plaque assay:

Two tenths ml aliquots of serially 10-fold diluted virus were inoculated onto confluent monolayer of cells in 35 mm plastic petridishes. After adsorption of the virus at 37°C for 1 hour, the monolayers were overlaid with 2 ml of MEM with 1% methylcellulose and 2% newborn calf serum. On day 3, the monolayers were stained with neutral red and plaque counts were made.

The results indicated that PPNa has no anti-HSV activity.

It can be concluded from above experiments that the activity of porphyrins used in the present invention against HIVs is specific.

## Claims

1. A use of the compound of the formula;

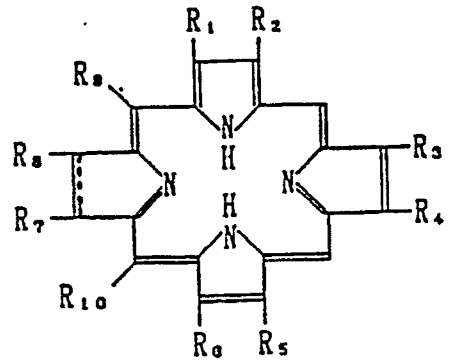

(I)

wherein either $R_1$, $R_2$, $R_3$, $R_4$, $R_5$, $R_6$, $R_7$ and $R_8$ are the same or different, each hydrogen atom, a lower alkyl group, a lower alkenyl group, a hydroxy(lower)alkyl group, a carboxy group or a carboxy(lower)alkyl group,
$R_9$ is hydrogen atom or a carboxy(lower)alkyl group,
with the proviso that at least one of $R_1$, $R_2$, $R_3$ $R_4$, $R_5$, $R_6$, $R_7$, $R_8$ and $R_9$ is a hydroxy(lower)alkyl group, a carboxy group or a carboxy(lower)alkyl group, and
$R_{10}$ is hydrogen atom or a lower alkyl group,
or $R_6$ and $R_{10}$ together form the group $-CO-CH_2-$ and $R_1$, $R_2$, $R_3$, $R_4$, $R_5$ ,$R_7$, $R_8$ and $R_9$ are as defined above, and
the symbol of a line and a dotted line means a single bond or a double bond,
an N-(lower)alkyl derivative, a metal coordination compound, a physiologically acceptable and hydrolyzable ester or a physiologically acceptable salt thereof for the manufacture of a medicament for treatment of diseases caused by HIVs.

2. The use according to Claim 1, in which either $R_1$, $R_2$, $R_3$, $R_4$, $R_5$, $R_6$, $R_7$ and $R_8$ are, the same or different, each hydrogen atom, methyl, ethyl, vinyl, 1-hydroxyethyl, 3-hydroxypropyl, carboxy, caboxymethyl or 2-carboxyethyl, $R_9$ is hydrogen atom or caboxymethyl, with the proviso that at least one of them is 1-hydroxyethyl, 3-hydroxypropyl, carboxy, caboxymethyl or 2-carboxyethyl, and $R_{10}$ is hydrogen atom or methyl,
or $R_6$ and $R_{10}$ together form the group $-CO-CH_2-$ and $R_1$, $R_2$ $R_3$, $R_4$ $R_5$ $R_7$, $R_8$ and $R_9$ are as defined above, and the symbol of a line and a dotted line is a single bond or a double bond, an N-methyl derivative or a physiologically acceptable salt thereof.

3. The use according to Claim 1, in which either
$R_1$ is methyl, carboxy, or carboxymethyl,
$R_2$ is hydrogen atom, methyl, ethyl, vinyl, 1-hydroxyethyl, or 2-carboxyethyl,
$R_3$ is methyl, ethyl, carboxymethyl or 2-carboxyethyl, $R_4$ is hydrogen atom, methyl, ethyl, vinyl, 1-hydroxyethyl, carboxymethyl or 2-carboxyethyl,
$R_5$ is methyl, vinyl, carboxymethyl or 2-carboxyethyl,
$R_6$ is hydrogen atom, methyl, 3-hydroxypropyl, carboxy, carboxymethyl or 2-carboxyethyl
$R_7$ is methyl, 3-hydroxypropyl, carboxymethyl or 2-carboxyethyl,
$R_8$ is methyl, carboxymethyl or 2-carboxyethyl,
$R_9$ is hydrogen atom or carboxymethyl, and
$R_{10}$ is hydrogen atom or methyl,
or $R_6$ and $R_{10}$ together form the group $-CO-CH_2-$ and $R_1$, $R_2$, $R_3$, $R_4$, $R_5$, $R_7$, $R_8$ and $R_9$ are as defined above.

4. The use according to Claim 1, in which the compound is selected from the group consisting of Hemin, Coproporphyrin I, Coproporphyrin II, Coproporphyrin III, Coproporphyrin III tetrasodium, Coproporphyrin III tetramethyl ester, Coproporphyrin IV, Deuteroporphyrin, Deuteroporphyrin dihydrochloride, Hematoporphyrin, Mesoporphyrin, Mesoporphyrin dihydrochloride, N-methyl Mesoporphyrin, Phylloporphyrin, Protoporphyrin, Protoporphyrin disodium, Protoporphyrin dimethyl ester, Di(decarboxyethyl)-di(3-hydroxypropyl)-Protoporphyrin, Pyrroporphyrin, Rhodoporphyrin, Uroporphyrin I, Uroporphyrin I dihydrochloride, Uroporphyrin II, Uroporphyrin III, Uroporphyrin IV, Phytoporphyrin and Chlorophyllin ligand.

5. The use according to Claim 1, in which the disease is selected from the group consisting of acquired immunodeficiency syndrome (AIDS), AIDS-related complex (ARC), persistent generalized lymphadenopathy (PGL) and lymphadenopathy syndrome (LAS).

6. The method according to Claim 1, in which the virus is selected from the group consisting of HTLV-III, LAV1, LAV2 and ARV.